# NOUVEAU FASCICULE DE BREVET EUROPEEN

(11) **EP 0 512 879 B2**
(45) Date de publication et mention de la décision concernant l'opposition: **04.06.2003**
(45) Mention de la délivrance du brevet: 28.12.1994
(21) Numéro de dépôt: 92401140.6
(22) Date de dépôt: 22.04.1992
(51) Int. Cl.: A61K 7/06, A61K 7/48, A61K 7/155, A61K 7/13, A61K 7/09

(54) **Composition cosmétique contenant un agent alcalinisant sans odeur**
Kosmetische Zusammensetzung, ein geruchloses Alkalinisierungsmittel enthaltend
Cosmetic composition containing odour-free alkalizing agent

(30) Priorité: 06.05.1991 FR 9105503
(43) Date de publication de la demande: 11.11.1992
(73) Titulaire: L'OREAL, 75008 Paris (FR)
(72) Inventeur: Samain, Henri, F-91570 Bièvres (FR); Dubief, Claude, F-78150 Le Chesnay (FR); Cotteret, Jean, F-78480 Verneuil S/Seine (FR)
(74) Mandataire: Peuscet, Jacques

(56) Documents cités:
- DE-A- 1 692 013
- DE-B- 1 035 856
- FR-A- 1 146 332
- GB-A- 2 188 948
- PATENT ABSTRACTS OF JAPAN, vol. 13, no. 84 (C-572), 27 février 1989;& JP-A-63 270 615 (TOYAMA CHEM. CO., LTD) 08-11-1988
- CHEMICAL ABSTRACTS, vol. 97, no. 25, 20 décembre 1982, page 254, abrégé no.209789m, Columbus, Ohio, US; H. RAUNIO et al.: "Independent induction and inhibition of ornithine decarboxylase and aryl hydrocarbon hydroxylase activities in rat epidermis", & J. Invest. Dermat. 1982, 79(4), 246-9

## Description

L'invention concerne une composition cosmétique comportant au moins un agent alcalinisant pratiquement sans odeur.

On sait que les compositions cosmétiques destinées au traitement des matières kératiniques humaines, telles que les cheveux, les poils ou la peau, contiennent, en général, au moins un agent alcalinisant destiné à amener le pH de la composition à une valeur généralement comprise entre 5 et 12,5. Dans le cas des compositions ayant un pH basique, le rôle de l'alcalinisant est différent selon le type de composition concerné : pour certaines compositions mettant en oeuvre de l'eau oxygénée comme oxydant, il faut amener l'eau oxygénée à pH basique pour permettre sa décomposition ; pour des compositions destinées à traiter les cheveux en général, l'adoption d'un pH basique permet de dilater la gaine cuticulaire des cheveux et facilite le passage des agents de traitement à l'intérieur de ladite gaine. Ces deux effets se retrouvent l'un ou l'autre, ou simultanément, dans la plupart des mises en oeuvre des compositions cosmétiques, qu'il s'agisse de dépilatoires, de compositions colorantes ou décolorantes pour cheveux, de compositions pigmentantes ou dépigmentantes pour la peau ou de compositions de déformation permanente des cheveux pour l'ondulation permanente ou le défrisage.

Dans l'état de la technique, les agents alcalinisants généralement utilisés dans les compositions cosmétiques, sont, soit l'ammoniaque, soit la monoéthanolamine. Dans les deux cas, ces agents ont une odeur désagréable, qui est gênante pour l'utilisateur. L'ammoniaque dégage une odeur forte, piquante, suffocante : elle est particulièrement incommodante dans les salons de coiffure pour les utilisateurs de produits de permanente, de coloration ou de décoloration. La monoéthanolamine ne permet, en outre, pas toujours d'atteindre des pH basiques relativement élevés. Il est un fait que, dans certaines des compositions cosmétiques susmentionnées, par exemple les compositions dépilatoires ou les compositions de permanente, on utilise des agents réducteurs qui ont, par eux-mêmes, une odeur assez désagréable ; néanmoins, l'utilisation d'agents d'alcalinisant malodorants ne fait que renforcer l'inconvénient olfactif susmentionné pour lesdites compositions.

Selon l'invention, on a constaté qu'il était possible d'utiliser, comme alcalinisants pour des compositions cosmétiques, certains diaminoalcanes, qui, non seulement, ont l'avantage de ne pas être malodorants par eux-mêmes, mais qui, en outre, ont l'avantage de donner des résultats complémentaires intéressants et inattendus ; lesdits résultats sont diversifiés suivant la destination des compositions cosmétiques en cause :
a) lorsqu'il s'agit d'une composition cosmétique destinée à l'ondulation permanente des cheveux, on a constaté qu'avec les alcalinisants proposés selon l'invention, la frisure était au moins aussi serrée que celle obtenue avec les compositions réductrices de l'art antérieur contenant de l'ammoniaque et était très nettement plus serrée que celle utilisant la monoéthanolamine ; en outre, les propriétés mécaniques des cheveux soumis à la permanente sont mieux préservées avec les alcalinisants proposés selon l'invention ; de plus, on a constaté que, lorsque la composition réductrice de la permanente contient de la cystéamine, on peut, en traitant les cheveux avec une solution contenant au moins un diaminoalcane, avant ou après l'application de la permanente ou entre les deux étapes de réduction et fixation de la permanente, combattre l'odeur désagréable résiduelle imprégnant les cheveux ;
b) lorsqu'il s'agit de compositions de coloration pour cheveux, on constate qu'avec les alcalinisants proposés selon l'invention, on obtient des teintures présentant l'avantage de moins dégrader la fibre capillaire, lesdites teintures pouvant être, dans certains cas, aussi puissantes que celles de l'art antérieur utilisant l'ammoniaque ;
c) lorsqu'il s'agit de compositions de décoloration des cheveux, ou éclaircissement de la couleur des cheveux, on a constaté qu'avec les alcalinisants proposés selon l'invention, elles présentent un pouvoir éclaircissant au moins égal à celui de compositions analogues alcalinisées à l'ammoniaque ;
d) lorsqu'il s'agit de compositions pour la dépilation, on a constaté que l'utilisation des alcalinisants proposés selon l'invention permet d'obtenir des compositions moins agressives vis-à-vis de la peau que celles renfermant de la monoéthanolamine.

La présente invention a donc pour but l'utilisation, dans des compositions cosmétiques en tant qu'agent alcalinisant inodore, d'une catégorie particulière de diaminoalcanes correspondant à la formule (I) ci-après définie.

La présente invention a pour objet une composition cosmétique destinée à être appliquée sur un élément kératinique humain, tel que la peau, les cheveux et les poils, comprenant, en milieu aqueux à pH basique, d'une part, un agent actif approprié pour ledit traitement et, d'autre part, un agent alcalinisant caractérisé par le fait que l'agent alcalinisant est constitué par au moins un composé de formule (I): 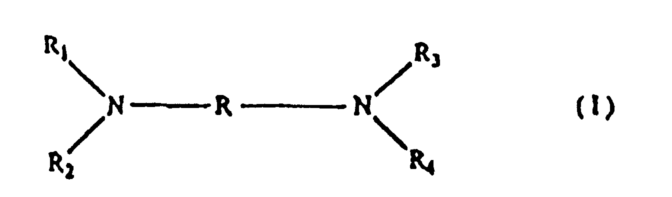 formule dans laquelle :
- R est un reste propylène éventuellement substitué par un groupement hydroxyle ou un radical alkyle C₁-C₄;
- R₁, R₂, R₃ et R₄, simultanément ou indépendamment l'un de l'autre, représentent l'hydrogène, un radical alkyle C₁-C₄ ou hydroxyalkyle (C₁-C₄).

Dans un mode préféré de réalisation, l'agent alcalinisant comprend au moins un composé choisi dans le groupe formé par le 1,3-diaminopropane, le N,N'-diéthyl 1,3-diaminopropane, le N,N-diéthyl 1,3-diaminopropane, le N,N-diméthyl 1,3-diaminopropane, le 2-hydroxy 1-(N,N-diéthyl)amino 3-aminopropane, le 2-hydroxy N,N'-ditertiobutyl 1,3-diaminopropane, le 2-hydroxy N,N'-tétraméthyl 1,3-diaminopropane et le 2-hydroxy 1,3-diaminopropane.

Dans une première variante de réalisation de l'invention, la composition cosmétique est destinée à être utilisée pour la déformation permanente des cheveux ou la dépilation ; dans ce cas, elle comprend, comme agent actif, un agent réducteur ; l'agent réducteur comprend, de préférence, au moins un composé choisi dans le groupe formé par l'acide thioglycolique, l'acide thiolactique, la cystéamine, la cystéine, l'hydroxy-4 N-(mercapto-2 éthyl) butyramide et les mercapto-4 butyramides N-mono ou N,N-disubstitués décrits dans la demande EP 638 763. Lorsqu'il s'agit d'une composition pour la déformation permanente des cheveux, le pH est généralement compris entre 5 et 10 ; lorsqu'il s'agit d'une composition dépilatoire, le pH est généralement compris entre 9 et 12,5.

Dans le cas de composition de permanente utilisant la cystéamine ou l'un de ses sels comme réducteur, l'application d'une solution aqueuse de diaminoalcanes de formule (I) en quantité suffisante pour que le pH soit supérieur à 7, et, de préférence supérieur à 8, avant ou après la permanente, ou entre les deux étapes réduction/fixation de la permanente, permet de combattre l'odeur désagréable résiduelle imprégnant les cheveux, particulièrement perceptible lorsque les cheveux traités sont à l'état mouillé ou dans un environnement humide. On préfère effectuer un prétraitement c'est-à-dire une application avant la permanente.

Dans une seconde variante, la composition selon l'invention est destinée à être utilisée pour la teinture des cheveux ; dans ce cas, elle comprend un agent actif constitué d'au moins un composé de coloration choisi dans le groupe formé par les précurseurs de colorant d'oxydation, les coupleurs, les colorants d'oxydation rapide et les précurseurs de pigments mélaniques ; le(s) composé(s) de coloration est (sont), de préférence, choisi(s) dans le groupe formé par les ortho- ou paraphénylènediamines, les ortho- ou paraaminophénols, les métadiamines aromatiques, les métaaminophénols, les métadiphénols, le 5,6-dihydroxyindole et ses dérivés ; le pH d'une telle composition est généralement ajusté entre 5 et 11.

Selon une troisième variante, la composition selon l'invention est destinée à la décoloration des cheveux ou à l'éclaircissement de la couleur des cheveux ; dans ce cas, la composition comprend, comme agent actif, un agent d'oxydation puissant ; le pH d'une telle composition est généralement compris entre 7 et 12.

Le pH des compositions selon l'invention est, suivant le type de composition concernée, obtenu en ajoutant des quantités plus ou moins importantes de diaminoalcanes de formule (I). Par exemple, dans les compositions destinées à l'ondulation permanente des cheveux, pour obtenir le pH souhaité par addition de 1,3-diaminopropane, on utilise généralement de 3,7 à 12 % en poids de l'agent alcalinisant, ce pourcentage étant donné par rapport au poids total de la composition. Lorsque la composition selon l'invention est destinée à la coloration ou à la décoloration des cheveux, le même agent alcalinisant que ci-dessus mentionné est utilisé dans des proportions comprises, de préférence, entre 4 et 10 % en poids par rapport au poids total de la composition. Les chiffres ci-dessus donnés ne sont aucunement limitatifs et dépendent essentiellement d'une part, de l'utilisation de la composition cosmétique en cause, d'autre part, de la nature des agents actifs ou des adjuvants présents dans la composition et enfin, du ou des composés alcalinisants choisis parmi l'ensemble des composés de formule (I).

Pour mieux faire comprendre l'objet de l'invention, on va décrire ci-après, à titre d'exemples purement illustratifs et non limitatifs, plusieurs modes de réalisation de l'invention.

### Exemples 1 à 6 :

On a formulé six compositions réductrices destinées à l'ondulation permanente des cheveux en utilisant, pour chacune d'elles, comme agent alcalinisant, un composé de formule (I). Les formulations ainsi réalisées sont données en grammes dans le tableau I ci-après :

**TABLEAU I**

| Exemple n° | 1 | 2 | 3 | 4 | 5 | 6 |
|---|---|---|---|---|---|---|
| Acide thioglycolique | 9,1 | 9,1 | 9,1 | 9,1 | | 9,1 |
| Acide thiolactique | | | | | 10,6 | |
| Chlorure d'oléocétyl diméthyl hydroxyéthyl ammonium en solution aqueuse à 30 % en poids de matières actives | 0,39 | 0,39 | 0,39 | 0,39 | 0,39 | 0,39 |
| 1,3-diaminopropane | 4,7 | 8,1 | | | 4,7 | |
| N,N-diméthyl 1,3-diaminopropane | | | 7,9 | | | |
| N,N-diéthyl 1,3-diaminopropane | | | | 8,6 | | |
| 2-hydroxy 1,3-diaminopropane | | | | | | 7,6 |
| Parfum, peptisant | qs | qs | qs | qa | qs | qs |
| pH | 8,5 | 9,5 | 8,5 | 8,5 | 8,5 | 8,5 |
| Eau qsp | 100 | 100 | 100 | 100 | 100 | 100 |

On constate tout d'abord que, pour chacune des compositions ci-dessus définies, l'odeur à l'utilisation est considérablement améliorée.

La mise en oeuvre de ces compositions est effectuée comme suit : chaque composition est appliquée sur des cheveux préalablement enroulés sur bigoudis. On laisse la composition agir pendant 15 minutes puis on rince à l'eau. On applique alors une solution oxydante d'eau oxygénée à 8 volumes et on laisse agir pendant 10 minutes. On rince ensuite à l'eau puis on déroule les cheveux. On constate que la frisure obtenue est excellente et que les cheveux sont en bon état. Pendant le traitement, aucune gêne n'a été provoquée par les odeurs provenant de l'agent alcalinisant.

### Exemple 7 :

On a réalisé une étude comparative de l'efficacité des compositions des exemples 1 à 6 pour la frisure des cheveux par rapport à des compositions correspondantes dans lesquelles l'agent alcalinisant utilisé était l'ammoniaque. Dans chaque cas, la quantité d'ammoniaque utilisée dans la composition comparative était celle nécessaire pour obtenir le pH indiqué pour chacune des formulations des exemples 1 à 6.

L'étude a été réalisée sur des perruques de cheveux humains ; les mèches ont une longueur égale à 15 cm. Les mèches de cheveux normalisées ont été enroulées sur des bigoudis de 9 mm de diamètre. L'application des compositions des exemples 1 à 6 et des compositions correspondantes réalisées avec de l'ammoniaque, a été effectuée suivant le processus décrit pour les exemples 1 à 6.

A la fin du traitement, on déroule les cheveux, et à mi-longueur des mèches humides, on mesure le rayon de courbure moyen des ondulations obtenues. L'efficacité de la composition testée est d'autant plus grande que le rayon de courbure est plus faible. Les résultats sont consignés dans le tableau II ci-après :

**TABLEAU II**

| N° de l'exemple | 1 | 2 | 3 | 4 | 5 | 6 |
|---|---|---|---|---|---|---|
| Nature de l'alcalinisant | | | | | | |
| Formule (I) | 0,66 | 0,50 | 0,68 | 0,88 | 3,50 | 0,66 |
| Ammoniaque | 1,16 | 0,57 | 1,16 | 1,16 | 6,00 | 1,16 |

On a constaté, par ailleurs, qu'en utilisant les agents alcalinisants de formule (I) on obtenait, par rapport à l'utilisation de l'ammoniaque, une amélioration des propriétés mécaniques des cheveux après traitement.

### Exemples 8 à 11 :

On a formulé 4 compositions pour la décoloration ou l'éclaircissement des cheveux en utilisant pour chacune d'elles, comme agent alcalinisant un composé de formule (I). Les formulations ainsi réalisées sont données en grammes dans le tableau III ci-après :

**TABLEAU III**

| EXEMPLE N° | 8 | 9 | 10 | 11 |
|---|---|---|---|---|
| 1,3-diaminopropane | 4,0 | - | - | - |
| 2-hydroxy 1,3-diaminopropane | - | 6,0 | - | - |
| N,N-diméthyl 1,3-diaminopropane | - | - | 4,0 | - |
| N,N-diéthyl 1,3-diaminopropane | - | - | - | 4,0 |
| Alcool oléique polyglycérolé à 2 moles de glycérol | 4,0 | 4,0 | 4,0 | 4,0 |
| Alcool oléique polyglycérolé à 4 moles de glycérol | 4,4 | 4,4 | 4,4 | 4,4 |
| Acide oléique | 3,0 | 3,0 | 3,0 | 3,0 |
| Amine oléique oxyéthylénée à 2 moles d'oxyde d'éthylène vendue par la Société "AKZO" sous la dénomination "ETHOMEEN 012" | 7,0 | 7,0 | 7,0 | 7,0 |
| Alcool oléique | 5,0 | 5,0 | 5,0 | 5,0 |
| Diéthanolamide oléique | 12,0 | 12,0 | 12,0 | 12,0 |
| Alcool éthylique | 10,0 | 10,0 | 10,0 | 10,0 |
| Monométhyléther du propylène glycol | 20,0 | 20,0 | 20,0 | 20,0 |
| Métabisulfite de sodium en solution aqueuse à 35 % | 0,46 | 0,46 | 0,46 | 0,46 |
| Agent séquestrant qs | | | | |
| Eau qsp | 100 | 100 | 100 | 100 |
| pH | 11,2 | 10,6 | 10,7 | 10,4 |

Au moment de l'emploi, les compositions des exemples 8 à 11 sont mélangées à un poids égal d'eau oxygénée à 20 volumes. Ces compositions sont appliquées sur des mèches de cheveux châtains. Après 30 minutes de pause, les cheveux sont rincés, lavés au shampooing et séchés.

Les cheveux traités avec la composition de l'exemple 8 passent du châtain au blond foncé ; ceux traités avec la composition des exemples 9, 10 et 11 passent du châtain au châtain clair.

On constate que, pour chacune des compositions définies ci-dessus, l'odeur à l'utilisation est considérablement améliorée.

### Exemple 12 : Exemple de coloration des cheveux

On procède à la coloration de cheveux gris à 90 % de blancs en appliquant la composition colorante d'oxydation suivante, contenant comme agent alcalinisant du 1,3-diaminopropane :

### ● Formulation colorante (A)

- Acide oléique (19 g) neutralisé par la monoéthanolamine (7,2 g) pour former un savon
- Alcool oléique 9 g
- Alcool éthylique 12,5 g
- Laurylsulfate de triéthanolamine à 28 % de matières actives (MA) 0,84 g MA
- Diéthanolamide oléique 8 g
- propylène glycol 10 g
- Alcool oléocéthylique oxyéthyléné à 30 moles d'oxyde d'éthylène 2 g
- Polymère cationique décrit et préparé selon le brevet français 2 270 846 constitué de motifs récurrents de formule : 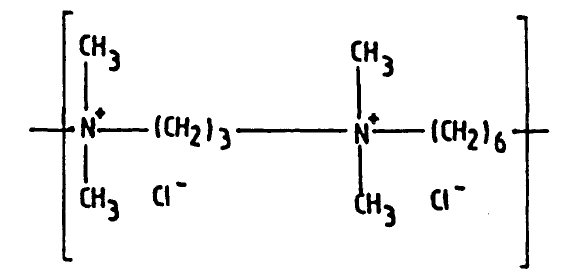 en solution à 60 % de MA 2,2 g MA
- Hydroquinone 0,15 g
- 1-phényl 3-méthyl 5-pyrazolone 0,2 g
- Métabisulfite de sodium en solution aqueuse à 35 % de MA 0,46 g MA
- 1,3-diaminopropane 8 g
- Agent séquestrant, parfum qs
   - Paraphénylènediamine 0,216 g
   - 6-(β-hydroxyéthyloxy) 1,3-diaminobenzène, 2HCl 0,482 g
   - Eau qsp 100 g
   - pH 11

### ● Formulation oxydante (B)

### - Eau oxygénée 20 volumes à pH = 3

Sur 3 g de cheveux, on applique 28 g du mélange poids pour poids des formulations (A) et (B). On laisse la composition agir pendant 30 minutes. On rince les cheveux à l'eau, on applique un shampooing et on sèche.

Les cheveux sont colorés en blond cendré bleuté ou blond foncé bleu cendré suivant qu'il s'agit respectivement de cheveux naturels ou permanentés.

On constate, qu'à l'utilisation, l'odeur de la composition colorante ci-dessus est considérablement améliorée.

### Exemple 13 :

On a préparé une composition dépilatoire contenant comme agent alcalinisant du 1,3-diamino propane ayant la composition suivante :

| • Partie a | |
|---|---|
| - Gluconate de calcium | 2,5 g |
| - Urée | 10 g |
| - Eau | 40 g |

| • Partie b | |
|---|---|
| - Alcool cétylstéarylique polyoxyéthyléné | 11 g |

| • Partie c | |
|---|---|
| - Carbonate de calcium | 6,15 g |

| • Partie d | |
|---|---|
| - Oxyde de calcium | 3,42 g |

### • Partie e

- Acide thioglycolique 4,23 g
- Eau 10 g
- 1,3-diamino propane qs pH = 8,25

On introduit à 75 ° C la partie b dans la partie a et on agite. On introduit la partie c dans le mélange (a + b) en maintenant sous agitation le mélange ainsi formé à 70°C pendant 20 minutes. On introduit la partie d dans le mélange (a + b + c) : après dissolution, on ramène la température à 20 ° C. La partie e est alors ajoutée. On complète à 100 g avec de l'eau.

On constate que, pour la composition ci-dessus, l'odeur est considérablement améliorée.

### Exemple 14 :

On effectue une permanente utilisant la cystéamine comme agent réducteur avec un prétraitement à l'aide d'un diaminopropane. On applique successivement sur les cheveux les compositions suivantes :

### • Composition A

- Acide acétique 6,1 g
- 1,3-diaminopropane qs pH 8,5
- Eau déminéralisée qsp 100 g

Cette composition est appliquée sur des cheveux naturels pendant 10 minutes. Après rinçage, on applique la composition B.

### • Composition B

- Chlorydrate de cystéamine 11,3 g
- Ammoniaque qs pH 8,5
- Eau déminéralisée qsp 100 g

Les cheveux sont enroulés sur des bigoudis. Après 15 minutes de pose, on rince et on applique la composition fixatrice C.

### • Composition C

- Eau oxygénée à 8 volumes 100 g
- Acide citrique qs pH 3,0

Après 5 minutes de pose, on rince et on déroule les cheveux.

Les mèches ainsi traitées sont laissées à l'air libre pendant 3 semaines à la température de 37°C. L'odeur se dégageant des mèches est évaluée par un panel de testeurs, 1 minute après vaporisation d'un nuage d'eau, comparativement à des mèches n'ayant subi que le traitement par les compositions B et C. A l'unanimité des testeurs, les mèches traitées avec les compositions B et C ont une odeur beaucoup plus désagréable que celles traitées avec les compositions A, B et C.

## Revendications

1. Composition cosmétique destinée à être appliquée sur un élément kératinique humain, tel que la peau, les cheveux et les poils, comprenant, en milieu aqueux, à un pH compris entre 5 et 12,5, d'une part, comme agent actif, un agent réducteur approprié pour la déformation permanente des cheveux ou la dépilation, un agent de coloration des cheveux choisi dans le groupe formé par les précurseurs de colorant d'oxydation, les coupleurs, les colorants d'oxydation rapide et les précurseurs de pigments mélaniques, ou un agent d'oxydation puissant approprié pour la décoloration et l'éclaircissement des cheveux et, d'autre part, un agent alcalinisant, **caractérisée par le fait que** l'agent alcalinisant est constitué par au moins un composé de formule (I): 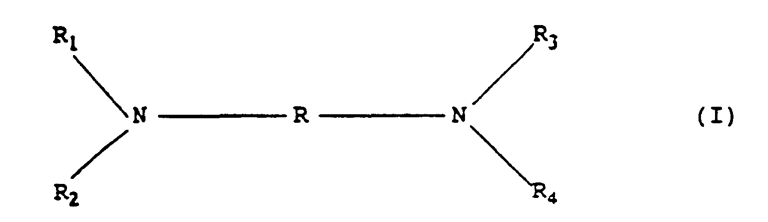 formule dans laquelle :
- R est un reste propylène éventuellement substitué par un groupement hydroxyle ou un radical alkyle C₁-C₄;
- R₁, R₂, R₃ et R₄, simultanément ou indépendamment l'un de l'autre, représentent l'hydrogène, un radical alkyle C₁-C₄ ou hydroxyalkyle (C₁-C₄).

2. Composition selon la revendication 1, **caractérisée par le fait que** l'agent alcalinisant comprend au moins un composé choisi dans le groupe formé par le 1,3-diaminopropane, le N,N-diéthyl 1,3-diaminopropane, le N,N'-diéthyl 1,3-diaminopropane, le N,N-diméthyl 1,3-diaminopropane, le 2-hydroxy 1-(N,N-diéthyl)amino 3-aminopropane, le 2-hydroxy N,N'-ditertiobutyl 1,3-diaminopropane, le 2-hydroxy N,N'-tétraméthyl 1,3-diaminopropane et le 2-hydroxy 1,3-diaminopropane.

3. Composition selon l'une des revendications 1 ou 2, **caractérisée par le fait que** l'agent réducteur comprend au moins un composé choisi dans le groupe formé par l'acide thioglycolique, l'acide thiolactique, la cystéamine, la cystéine, l'hydroxy-4 N-(mercapto-2 éthyl) butyramide et les mercapto-4 butyramides N-mono ou N,N-disubstitués.

4. Composition selon la revendication 3, destinée à être utilisée pour la déformation permanente des cheveux, **caractérisée par le fait que** son pH est compris entre 5 et 10.

5. Composition selon la revendication 3, destinée à être utilisée comme dépilatoire, **caractérisée par le fait que** son pH est compris entre 9 et 12,5.

6. Composition selon la revendication 1, **caractérisée par le fait que** l'agent de coloration comporte au moins un composé choisi dans le groupe formé par les ortho- ou paraphénylènediamines, les ortho- ou paraaminophénols, les métadiamines aromatiques, les métaaminophénols, les métadiphénols, le 5,6-dihydroxyindole et ses dérivés.

7. Composition selon l'une des revendications 1 ou 6, **caractérisée par le fait que** son pH est compris entre 5 et 11.

8. Composition selon l'une des revendications 1 ou 2, destinée à être utilisée pour la décoloration et l'éclaircissement des cheveux, **caractérisée par le fait que** son pH est compris entre 7 et 11.

9. Procédé pour traiter des cheveux, dans lequel on fait agir sur lesdits cheveux un agent actif approprié pour un traitement de permanente mettant en oeuvre la cystéamine, **caractérisé par le fait qu'**on applique sur les cheveux au moins un diaminoalcane de formule (I) avant ou après application de la permanente ou entre les deux étapes de réduction et de fixation de la permanente.

## Patentansprüche

1. Kosmetische Zusammensetzung zur Auftragung auf ein menschliches keratinhaltiges Element, wie der Haut, dem Kopfhaar und dem Barthaar, umfassend in wässrigem Milieu bei einem pH im Bereich von 5 bis 12,5 als aktives Mittel ein zur Dauerwellung des Kopfhaars oder Enthaarung geeignetes Reduktionsmittel, ein Haarfärbemittel, welches ausgewählt ist unter Oxidationsfarbstoff-Vorläufem, Kupplem, Schnell-Oxidationsfarbstoffen und Präkursoren von Melaminpigmenten, oder ein zur Entfärbung und Aufhellung von Kopfhaar geeignetes kräftiges Oxidationsmittel und ein alkalinisierendes Mittel, **dadurch gekennzeichnet, dass** das alkalinisierende Mittel aus wenigstens einer Verbindung der Formel (I) besteht: 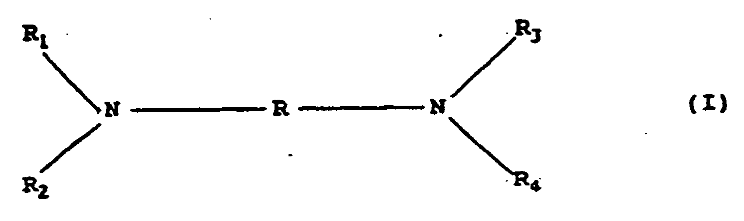 worin:
- R für einen Propylenrest steht, der gegebenenfalls mit einer Hydroxylgruppe oder einem C₁-C₄-Alkylrest substituiert ist; und
- R₁, R₂, R₃ und R₄ gleichzeitig oder unabhängig voneinander für ein Wasserstoffatom, einen C₁-C₄-Alkylrest oder einen Hydroxy-(C₁-C₄)-alkylrest stehen.

2. Zusammensetzung nach Anspruch 1, **dadurch gekennzeichnet, dass** das alkalinisierende Mittel wenigstens eine Verbindung umfasst, die ausgewählt ist unter 1,3-Diaminopropan, N,N-Diethyl-1,3-diaminopropan, N,N'-Diethyl-1,3-diaminopropan, N,N-Dimethyl-1,3-diaminopropan, 2-Hydroxy-1-(N,N-diethyl)amino-3-aminopropan, 2-Hydroxy-N,N'-ditert.-butyl-1,3-diaminopropan, 2-Hydroxy-N,N'-tetramethyl-1,3-diaminopropan und 2-Hydroxy-1,3-diaminopropan.

3. Zusammensetzung nach Anspruch 1 oder 2, **dadurch gekennzeichnet, dass** das Reduktionsmittel wenigstens eine Verbindung umfasst, die ausgewählt ist unter Thioglykolsäure, Thiomilchsäure, Cysteamin, Cystein, 4-Hydroxy-N-(2-mercaptoethyl)butyramid und 4-Mercaptobutyramiden, die N-mono- oder N,N-disubstituiert sind.

4. Zusammensetzung nach Anspruch 3 zur Verwendung bei der Dauerwellbehandlung des Kopfhaars, **dadurch gekennzeichnet, dass** deren pH im Bereich von 5 bis 10 liegt.

5. Zusammensetzung nach Anspruch 3 zur Verwendung als Enthaarungsmittel, **dadurch gekennzeichnet, dass** deren pH im Bereich von 9 bis 12,5 liegt.

6. Zusammensetzung nach Anspruch 1, **dadurch gekennzeichnet, dass** das Färbemittet wenigstens eine Verbindung umfasst, die ausgewählt ist unter Ortho- oder Paraphenylendiaminen, Ortho- oder Paraaminophenolen, aromatischen Metadiaminen, Metaaminophenolen, Metadiphenolen, 5,6-Dihydroxyindol und Derivaten davon.

7. Zusammensetzung nach einem der Ansprüche 1 oder 6, **dadurch gekennzeichnet, dass** deren pH-Wert im Bereich von 6 bis 11 liegt.

8. Zusammensetzung nach Anspruch 1 oder 2 zur Entfärbung und Aufhellung von Kopfhaar, **dadurch gekennzeichnet, dass** deren pH im Bereich von 7 bis 11 liegt.

9. Verfahren zur Behandlung von Kopfhaar, wobei man auf das Kopfhaar ein zur Dauerwellbehandlung geeignetes aktives Mittel unter Verwendung von Cysteamin einwirken lässt, **dadurch gekennzeichnet, dass** man auf das Kopfhaar wenigstens ein Diaminoalkan der Formal (I) vor oder nach dem Aufbringen der Dauerwelle oder zwischen den beiden Stufen der Reduktion und der Fixierung der Dauerwelle aufträgt.

## Claims

1. Cosmetic composition intended to be applied to a human keratinous organ such as the skin, hair and body hair, comprising, in an aqueous medium at a pH of between 5 and 12.5, on the one hand, as active agent a reducing agent suitable for the permanent deformation of hair or hair removal, a hair-dyeing agent chosen from the group consisting of oxidation dye precursors, couplers, rapid oxidation dyes and melanic pigment precursors, or a powerful oxidizing agent suitable for bleaching and lightening hair and, on the other hand, an alkalifying agent, **characterized in that** the alkalifying agent consists of at least one compound of formula (I): 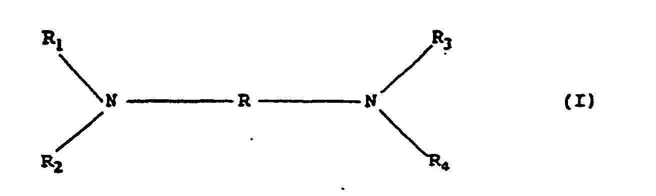 in which formula:
- R is a propylene residue optionally substituted by a hydroxyl group or a C₁-C₄ alkyl radical;
- R₁, R₂, R₃ and R₄, simultaneously or independently of each other, denote hydrogen or a C₁-C₄ alkyl or hydroxyalkyl (C₁-C₄) radical.

2. Composition according to Claim 1, **characterized in that** the alkalifying agent comprises at least one compound chosen from the group consisting of 1,3-diaminopropane, N,N-diethyl-1,3-diaminopropane, N,N'-diethyl-1, 3-diaminopropane, N,N-dimethyl-1,3-diaminopropane, 2-hydroxy-1-(N,N-diethyl)amino-3-aminopropane, 2-hydroxy-N,N'-di-tert-butyl-1,3-diaminopropane, 2-hydroxy-N,N'-tetramethyl-1,3-diaminopropane and 2-hydroxy-1,3-diaminopropane.

3. Composition according to either of Claims 1 and 2, **characterized in that** the reducing agent comprises at least one compound chosen from the group consisting of thioglycolic acid, thiolactic acid, cysteamine, cysteine, 4-hydroxy-N-(2-mercaptoethyl)-butyramide and N-mono- or N,N-disubstituted 4-mercaptobutyramides.

4. Composition according to Claim 3, intended to be employed for the permanent deformation of hair, **characterized in that** its pH is between 5 and 10.

5. Composition according to Claim 3, intended to be employed as a hair-remover, **characterized in that** its pH is between 9 and 12.5.

6. Composition according to Claim 1, **characterized in that** the dyeing agent comprises at least one compound chosen from the group consisting of ortho- or para-phenylenediamines, ortho- or para-aminophenols, aromatic meta-diamines, meta-aminophenols, meta-diphenols, 5,6-dihydroxyindole and its derivatives.

7. Composition according to either of Claims 1 and 6, **characterized in that** its pH is between 5 and 11.

8. Composition according to either of Claims 1 and 2, intended to be employed for bleaching and lightening hair, **characterized in that** its pH is between 7 and 11.

9. Method for treating hair, in which an active agent suitable for a permanent waving treatment using cysteamine is allowed to act on the said hair, **characterized in that** at least one diaminoalkane of formula (I) is applied to the hair before or after applying the permanent waving or between the two reducing and fixing stages of the permanent waving.
